(19) Europäisches Patentamt
European Patent Office
Office européen des brevets

(11) Veröffentlichungsnummer: **0 241 826**
**A1**

(12) **EUROPÄISCHE PATENTANMELDUNG**

(21) Anmeldenummer: **87105007.6**

(22) Anmeldetag: **04.04.87**

(51) Int. Cl.[4]: **C07C 149/243** , C07C 149/20 , A01N 37/44

(30) Priorität: **17.04.86 DE 3612941**
**08.11.86 DE 3638151**

(43) Veröffentlichungstag der Anmeldung:
**21.10.87 Patentblatt 87/43**

(84) Benannte Vertragsstaaten:
**AT BE CH DE ES FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Kluth, Joachim, Dr.**
**Kurt-Schumacher-Strasse 9**
**D-4018 Langenfeld(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch Gladbach 2(DE)**

(54) **3-Amino-2-cyano-acrylsäureester.**

(57) Die Erfindung betrifft neue 3-Amino-2-cyano-acrylsäureester der Formel (I)

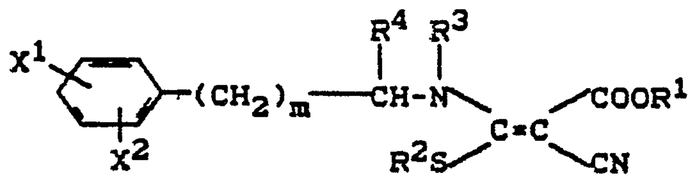

(I)

in welcher
$R^1$ für Alkoxyalkyl oder Benzyloxyalkyl steht,
$R^2$ für Alkyl oder Alkenyl steht,
$R^3$ für Wasserstoff oder Alkyl steht,
$R^4$ für Wasserstoff oder für gegebenenfalls durch Hydroxy, Halogen, Alkoxy oder Dialkylamino substituiertes Alkyl steht,
$X^1$ und $X^2$ gleich oder verschieden sind und für Wasserstoff, Halogen, Nitro, Alkyl, Alkoxy, Alkylthio, Halogenalkyl, Halogenalkoxy, Amino, Alkylamino, Alkylcarbonylamino, N-Alkylcarbonyl-N-alkylamino, Dialkylaminocarbonylamino oder jeweils gegebenenfalls substituiertes Aryl oder Aryloxy stehen, oder
$X^1$ und $X^2$ gemeinsam mit dem angerenzenden Phenylrest für Naphthyl stehen und
m für die Zahlen 0, 1, 2, 3, 4 oder 5 steht,
für den Fall, daß $R^4$ nicht für Wasserstoff steht, auch deren optisch aktive Verbindungen,
ein Verfahren zu ihrer Herstellung und ihre Verwendung als Herbizide sowie neue Zwischenprodukte.

EP 0 241 826 A1

# 3-Amino-2-cyano-acrylsäureester

Die Erfindung betrifft neue 3-Amino-2-cyano-acrylsäureester, ein Verfahren zu ihrer Herstellung und ihre Verwendung als Herbizide.

Es ist bereits bekannt, daß bestimmte Aralkylaminoacrylnitrile herbizide Eigenschaften aufweisen (vgl. z.B. US-PS 4 154 599 und 4 201 569). So kann zum Beispiel 2-Cyano-3-(1-phenylethylamino)-3-methylthioacrylsäuremethylester zur Unkrautbekämpfung eingesetzt werden. Die Wirkung dieses Stoffes ist gut, jedoch werden bei geringen Aufwandmengen einige Unkräuter nicht immer voll erfaßt, außerdem ist die Selektivität nicht immer zufriedenstellend.

Es werden nun neue 3-Amino-2-cyano-acrylsäureester der Formel (I)

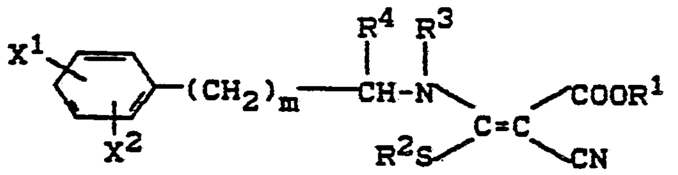

(I)

in welcher
$R^1$ für Alkoxyalkyl oder Benzyloxyalkyl steht,
$R^2$ für Alkyl oder Alkenyl steht,
$R^3$ für Wasserstoff oder Alkyl steht,
$R^4$ für Wasserstoff oder für gegebenenfalls durch Hydroxy, Halogen, Alkoxy oder Dialkylamino substituiertes Alkyl steht,
$X^1$ und $X^2$ gleich oder verschieden sind und für Wasserstoff, Halogen, Nitro, Alkyl, Alkoxy, Alkylthio, Halogenalkyl, Halogenalkoxy, Amino, Alkylamino, Alkylcarbonylamino, N-Alkylcarbonyl-N-alkylamino, Dialkylaminocarbonylamino oder jeweils gegebenenfalls substituiertes Aryl oder Aryloxy stehen, oder
$X^1$ und $X^2$ gemeinsam mit dem angrenzenden Phenylrest für Naphthyl stehen und
m für die Zahlen 0, 1, 2, 3, 4 oder 5 steht,
gefunden.

Die 3-Amino-2-cyano-acrylsäureester der Formel (I) können in der E-oder Z-Form vorliegen. Vorwiegend fallen sie als Gemische beider Formen (E/Z) an.

Für den Fall, daß $R^4$ nicht für Wasserstoff steht, enthalten die 3-Amino-2-cyano-acrylsäureester der Formel (I) mindestens ein asymmetrisch substituiertes Kohlenstoffatom und können deshalb in verschiedenen enantiomeren Formen vorliegen. Die Erfindung betrifft sowohl die möglichen einzelnen Isomeren als auch Gemische dieser Isomeren.

Weiterhin wurde gefunden, daß man die 3-Amino-2-cyano-acrylsäureester der Formel (I) erhält, wenn man 2-Cyano-acrylsäureester der Formel (II)

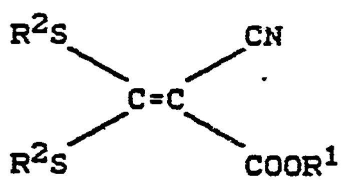

(II)

in welcher
$R^1$ und $R^2$ die oben angegebenen Bedeutungen haben,
mit Aminen der Formel (III)

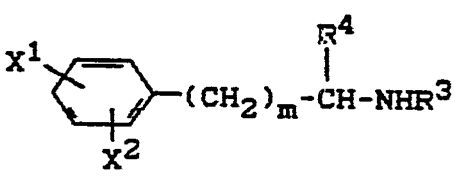

(III)

in welcher

m, $X^1$, $X^2$, $R^3$ und $R^4$ die oben angegebenen Bedeutungen haben, gegebenenfalls in Gegenwart von Verdünnungsmitteln umsetzt.

Schließlich wurde gefunden, daß sich die neuen 3-Amino-2-cyano-acrylsäureester der Formel (I) durch eine hervorragende herbizide Wirkung auszeichnen.

Überraschenderweise besitzen die erfindungsgemäßen 3-Amino-2-cyano-acrylsäureester der Formel (I) eine wesentlich bessere herbizide Wirksamkeit als der 2-Cyano-3-(1-phenylethylamino)-3-methylthio-acrylsäuremethylester, welcher ein konstitutionell ähnlicher Wirkstoff gleicher Wirkungsrichtung ist.

Die erfindungsgemäßen 3-Amino-2-cyano-acrylsäureester sind durch die Formel (I) allgemein definiert. Bevorzugt sind diejenigen Verbindungen der Formel (I), in denen

$R^1$ für Alkoxyalkyl mit jeweils 1 bis 6 Kohlenstoffatomen in den einzelnen Alkylteilen oder für Benzyloxyalkyl mit 1 bis 6 Kohlenstoffatomen im Alkylteil steht,

$R^2$ für Alkyl mit 1 bis 6 Kohlenstoffatomen oder für Alkenyl mit 3 bis 8 Kohlenstoffatomen steht,

$R^3$ für Wasserstoff oder Alkyl mit 1 bis 6 Kohlenstoffatomen steht,

$R^4$ für Wasserstoff oder für gegebenenfalls durch Hydroxy, Fluor, Chlor, $C_1$-$C_4$-Alkoxy oder Di-$C_1$-$C_4$-alkylamino substituiertes Alkyl mit 1 bis 6 Kohlenstoffatomen steht,

$X^1$ und $X^2$ gleich oder verschieden sind und für Wasserstoff, Fluor, Chlor, Brom, Nitro, $C_1$-$C_4$-Alkyl, $C_1$-$C_4$-Alkoxy, $C_1$-$C_4$-Alkylthio, Halogen-$C_1$-$C_4$-alkyl, Halogen-$C_1$-$C_4$-alkoxy, Amino, $C_1$-$C_4$-Alkylamino, $C_1$-$C_4$-Alkylcarbonylamino, N-$C_1$-$C_4$-Alkylcarbonyl-N-$C_1$-$C_4$-alkylamino, Di-$C_1$-$C_4$-alkylaminocarbonylamino oder für jeweils gegebenenfalls einfach bis dreifach, gleich oder verschieden durch Halogen, wie Fluor, Chlor und Brom, Nitro, Cyano, $C_1$-$C_2$-Alkyl, $C_1$-$C_2$-Alkoxy, $C_1$-$C_2$-Alkylthio, Trifluormethyl, Trifluormethoxy oder Trifluormethylthio substituiertes Aryl oder Aryloxy mit jeweils 6 bis 10 Kohlenstoffatomen im Arylteil stehen, oder

$X^1$ und $X^2$ gemeinsam mit dem angrenzenden Phenylrest für Naphthyl stehen und

m für die Zahlen 0, 1, 2 oder 3 steht.

Besonders bevorzugt sind diejenigen Verbindungen der Formel (I), in denen

$R^1$ für Alkoxyalkyl mit jeweils 1 bis 4 Kohlenstoffatomen in den einzelnen Alkylteilen oder für Benzyloxyalkyl mit 1 bis 4 Kohlenstoffatomen im Alkylteil steht,

$R^2$ für Alkyl mit 1 bis 4 Kohlenstoffatomen oder Alkenyl mit 3 bis 6 Kohlenstoffatomen steht,

$R^3$ für Wasserstoff oder Alkyl mit 1 bis 4 Kohlenstoffatomen steht,

$R^4$ für Wasserstoff oder für gegebenenfalls durch Hydroxy, Fluor, Chlor, Methoxy, Ethoxy, Dimethylamino oder Diethylamino substituiertes Alkyl mit 1 bis 4 Kohlenstoffatomen steht,

$X^1$ und $X^2$ gleich oder verschieden sind und für Wasserstoff, Fluor, Chlor, Brom, Nitro, Methyl, Ethyl, Methoxy, Ethoxy, Methylthio, Ethylthio, Trifluormethyl, Trifluormethoxy, Amino, Methylamino, Ethylamino, n-Propylamino, i-Propylamino, n-Butylamino, tert.-Butylamino, Methylcarbonylamino, Ethylcarbonylamino, n-Propylcarbonylamino, i-Propylcarbonylamino, n-Butylcarbonylamino, i-Butylcarbonylamino, tert.-Butylcarbonylamino, N-Methylcarbonyl-N-methylamino, N-Ethylcarbonyl-N-methylamino, N-n-Propylcarbonyl-N-methylamino, N-i-Propylcarbonyl-N-methylamino oder für jeweils gegebenenfalls einfach oder zweifach, gleich oder verschieden durch Fluor, Chlor, Nitro, Cyano, Methyl, Ethyl, Methoxy, Ethoxy, Methylthio, Ethylthio, Trifluormethyl, Trifluormethoxy oder Trifluormethylthio substituiertes Phenyl oder Phenoxy stehen, oder

$X^1$ und $X^2$ gemeinsam mit dem angrenzenden Phenylrest für Naphthyl stehen und

m für die Zahlen 0, 1 oder 2 steht.

Ganz besonders bevorzugt sind diejenigen Verbindungen der Formel (I), in denen

$R^1$ für Methoxyethyl, Ethoxyethyl, n-Propoxyethyl, i-Propoxyethyl, n-Butoxyethyl, i-Butoxyethyl, sec.-Butoxyethyl, Benzyloxymethyl oder Benzyloxyethyl steht,

$R^2$ für Methyl, Ethyl, n-Propyl, i-Propyl, n-Butyl, i-Butyl oder sec.-Butyl, 1-Propen-3-yl oder 2-Penten-4-yl steht,

$R^3$ für Wasserstoff, Methyl, Ethyl, n-Propyl, i-Propyl, n-Butyl, i-Butyl oder sec.-Butyl steht,

$R^4$ für Wasserstoff, Methyl, Ethyl, n-Propyl, i-Propyl, n-Butyl, i-Butyl oder sec.-Butyl steht,

$X^1$ und $X^2$ gleich oder verschieden sind und für Wasserstoff, Fluor, Chlor, Nitro, Methyl, Methoxy, Methylthio, Trifluormethyl, Trifluormethoxy, Methylamino, Ethylamino, Methylcarbonylamino, Ethylcarbonylamino oder für jeweils gegebenenfalls einfach oder zweifach, gleich oder verschieden durch Fluor, Chlor, Nitro, Cyano, Methyl, Methoxy, Methylthio, Trifluormethyl, Trifluormethoxy oder Trifluormethylthio substituiertes Phenyl oder Phenoxy stehen, oder

$X^1$ und $X^2$ gemeinsam mit dem angrenzenden Phenylrest für Naphthyl stehen und

m für die Zahlen 0 oder 1 steht.

Bervorzugt sind außerdem die entsprechenden geometrischen und optischen Isomeren der Verbindungen der Formel (I).

Besonders bevorzugte optische Isomeren sind die R-und S-Enantiomeren der Verbindungen der Formel (Ib)

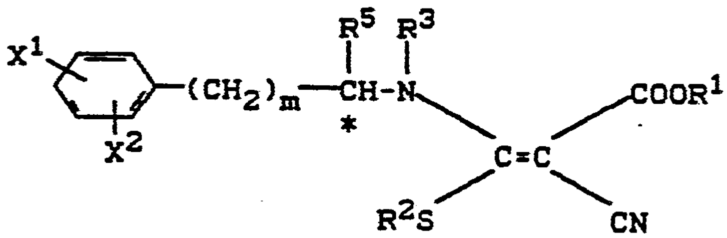

(Ib)

in welcher
$X^1$, $X^2$, $R^1$, $R^2$, $R^3$ und m die oben angegebenen vorzugsweisen Bedeutungen haben und in denen das mit dem Stickstoff verbundene, durch (*) gekennzeichnete Kohlenstoffatom ein Chiralitätszentrum darstellt und $R^5$ für $C_1$-$C_4$-Alkyl steht.

Ganz besonders bevorzugt sind die S-Enantiomeren der Verbindungen der Formel (Ib), in welcher
$X^1$, $X^2$, $R^1$, $R^2$, $R^3$ und m die oben angegebenen besonders bevorzugten Bedeutungen haben und $R^5$ für Methyl steht.

Verwendet man 2-Cyano-3,3'-diethylthio-acrylsäure-methoxyethylester und 1-Phenylethylamin als Ausgangsstoffe, so kann der Verlauf des erfindungsgemäßen Verfahren durch das folgende Formelschema wiedergegeben:

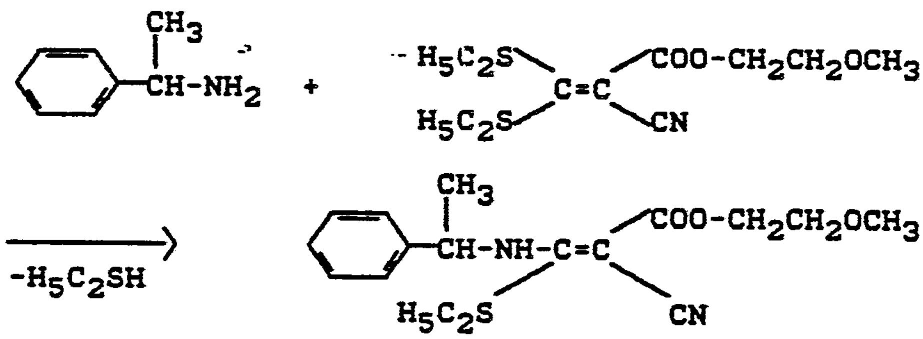


Die beim erfindungsgemäßen Verfahren als Ausgangsstoffe zu verwendenden 2-Cyano-acrylsäureester sind durch die Formel (II) allgemein definiert. In Formel (II) haben $R^1$ und $R^2$ vorzugsweise bzw. insbesondere die gleichen Bedeutungen, wie sie oben im Rahmen der Substituentendefinition der Formel (I) vorzugsweise bzw. als insbesondere bevorzugt angegeben sind.

Als Beispiele für die Verbindungen der Formel (II) seien genannt:

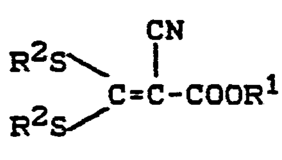

(II)

Tabelle 1

| $R^1$ | $R^2$ |
|---|---|
| $-CH_2CH_2OCH_3$ | $CH_3$ |
| $-CH_2CH_2OC_2H_5$ | $CH_3$ |
| $-CH_2CH_2OC_3H_7$-n | $CH_3$ |
| $-CH_2CH_2OC_3H_7$-i | $CH_3$ |
| $-CH_2CH_2OC_4H_9$-n | $CH_3$ |
| $-CH_2CH_2OC_4H_9$-i | $CH_3$ |
| $-CH_2CH_2OC_4H_9$-sec. | $CH_3$ |
| $-CH_2CH_2OC_4H_9$-tert. | $CH_3$ |
| $-CH_2CH_2OCH_3$ | $C_2H_5$ |
| $-CH_2CH_2OC_2H_5$ | $C_2H_5$ |
| $-CH_2CH(CH_3)OC_2H_5$ | $C_2H_5$ |
| $-CH_2CH_2OC_3H_7$-n | $C_2H_5$ |
| $-CH_2CH_2OC_3H_7$-i | $C_2H_5$ |
| $-CH_2CH_2OCH_3$ | n-$C_3H_7$ |
| $-CH_2CH_2OC_2H_5$ | n-$C_3H_7$ |
| $-CH_2CH(CH_3)OC_2H_5$ | n-$C_3H_7$ |
| $-CH_2CH_2OC_3H_7$-n | n-$C_3H_7$ |
| $-CH_2CH_2OC_3H_7$-i | n-$C_3H_7$ |
| $-CH_2CH_2OCH_3$ | i-$C_3H_7$ |

**Tabelle 1 - Fortsetzung**

| $R^1$ | $R^2$ |
|---|---|
| $-CH_2CH_2OC_2H_5$ | $i-C_3H_7$ |
| 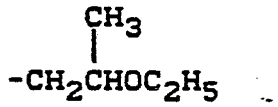 | $i-C_3H_7$ |
| $-CH_2CH_2OC_3H_7-n$ | $i-C_3H_7$ |
| $-CH_2CH_2OC_3H_7-i$ | $i-C_3H_7$ |
| 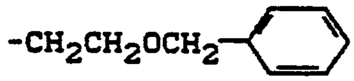 | $CH_3$ |
| 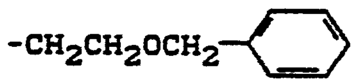 | $C_2H_5$ |
| 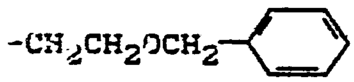 | $n-C_3H_7$ |
| 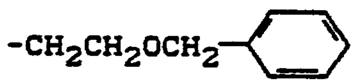 | $i-C_3H_7$ |

Die Verbindungen der Formel (II) sind neu und lassen sich auf einfache Weise nach bekannten Methoden herstellen (vgl. Chem. Ber. 95 (1962), 2861 - 2869). Man erhält die Verbindungen der Formel (II), wenn man 2-Cyanoessigsäureester der Formel (IV)

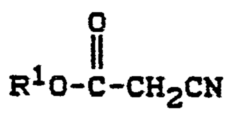 (IV)

in welcher
$R^1$ die oben angegebenen Bedeutungen hat,
mit Schwefelkohlenstoff in Gegenwart von mindestens der zweifachen molaren Menge Alkalialkoholat, wie z. B. $NaOR^1$, $KOR^1$ oder Natrium-und Kalium-tert.-butanolat und in Gegenwart von inerten Verdünnungsmitteln wie z. B. Diethylether, Dioxan, Tetrahydrofuran oder Alkohole der Formel $R^1OH$ bei Temperaturen zwischen -10 °C und +60 °C, vorzugsweise zwischen 0 °C und + 40 °C zu den neuen Salzen der Formel (V)

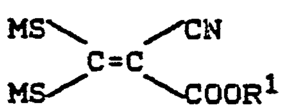 (V)

in welcher
$R^1$ die oben angegebenen Bedeutungen hat und
M für ein Alkalimetallatom steht,
umsetzt und anschließend diese Verbindungen der Formel (V) gegebenenfalls nach ihrer Isolierung, mit Alkylierungsmitteln der Formel (VI)
$R^2Q$ (VI)

in welcher
$R^2$ die oben angegebenen Bedeutungen hat und
Q für Halogen oder für den Rest $-OSO_2OR^2$ steht,
in Gegenwart von inerten Verdünnungsmittel wie z. B. Wasser, Wasser/Aceton-Gemisch, Ether, wie z. B. Diethylether, Di-n-butylether, Tetrahydrofuran oder Dioxan bei Temperaturen zwischen 0 °C und 80 °C, vorzugsweise zwischen +15 °C und +50 °C umsetzt.

Die für die Herstellung der neuen Verbindungen der Formel (II) zu verwendenden 2-Cyanoessigsäureester sind durch die Formel (IV) allgemein definiert. In dieser Formel (IV) steht $R^1$ vorzugsweise bzw. besonders bevorzugt für diejenigen Reste, die bereits im Zusammenhang mit der Beschreibung der erfindungsgemäßen Stoffe der Formel (I) vorzugsweise bzw. insbesondere bevorzugt für diesen Substituenten genannt wurden.

Die 2-Cyanoessigsäureester der Formel (IV) sind bekannt und/oder lassen sich nach bekannten Methoden herstellen (vgl z.B. WO-PS 85/00598).

Als Beispiele für die Verbindungen der Formel (IV) seien genannt:

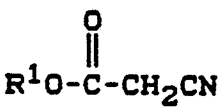 (IV)

__Tabelle 2__

| $R^1$ | $R^1$ | $R^1$ |
|---|---|---|
| $-CH_2CH_2OCH_3$ | $-CH_2CH_2OC_4H_9$-i | $-CH_2CH_2CH_2-OCH_3$ |
| $-CH_2CH_2OC_2H_5$ | $-CH_2CH_2OC_4H_9$-sec. | $-CH_2CH_2CH_2-OC_2H_5$ |
| $-CH_2CH_2OC_3H_7$-n | $-CH_2CH_2OC_4H_9$-tert. | $-CH_2CH(CH_3)OCH_3$ |
| $-CH_2CH_2OC_3H_7$-i | 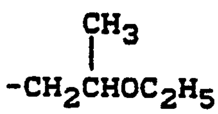 | $-CH_2CH_2OCH_2-$ 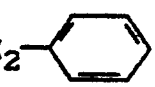 |
| $-CH_2CH_2OC_4H_9$-n | $-CH_2CH_2OCH_2CH=CH_2$ | |

Die für die Herstellung der neuen Verbindungen der Formel (II) außerdem als Ausgangstoffe zu verwendenden Salze sind durch die Formel (V) allgemein definiert. In dieser Formel (V) steht $R^1$ vorzugsweise bzw. besonders bevorzugt für diejenigen Reste, die bereits im Zusammenhang mit der Beschreibung der erfindungsgemäßen Stoffe der Formel (I) vorzugsweise bzw. insbesondere bevorzugt für diesen Substituenten genannt wurden. M steht in dieser Formel für ein Alkalimetallatom, vorzugsweise für ein Natrium-oder Kaliumatom.

Die Herstellung der neuen Verbindungen der Formel (V) wird nach dem oben angegebenen Verfahren durchgeführt.

Als Beispiele für die Verbindungen der Formel (V) seien genannt:

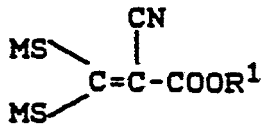

(V)

M = Natrium oder Kalium

Tabelle 3

| $R^1$ | $R^1$ | $R^1$ |
|---|---|---|
| $-CH_2CH_2OCH_3$ | $-CH_2CH_2OC_4H_9$-i | $-CH_2CH_2CH_2-OCH_3$ |
| $-CH_2CH_2OC_2H_5$ | $-CH_2CH_2OC_4H_9$-sec. | $-CH_2CH_2CH_2-OC_2H_5$ |
| $-CH_2CH_2OC_3H_7$-n | $-CH_2CH_2OC_4H_9$-tert. | $-CH_2CH(CH_3)OCH_3$ |
| $-CH_2CH_2OC_3H_7$-i | 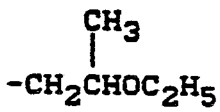 | $CH_2CH_2OCH_2$- 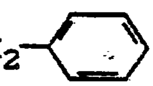 |
| $-CH_2CH_2OC_4H_9$-n | $-CH_2CH_2OCH_2CH=CH_2$ | |

Die weiterhin für die Herstellung der neuen Verbindungen der Formel (II) als Ausgangstoffe zu verwendenden Alkylierungsmittel sind durch die Formel (VI) allgemein definiert. In dieser Formel (VI) steht $R^2$ vorzugsweise bzw. besonders bevorzugt für diejenigen Reste, die bereits im Zusammenhang mit der Beschreibung der erfindungsgemäßen Stoffe der Formel (I) vorzugsweise bzw. insbesondere bevorzugt für diesen Substituenten genannt wurden. Q steht in dieser Formel (VI) für Halogen, vorzugsweise Chlor, Brom oder Iod sowie für den Rest $-OSO_2OR^2$, worin $R^2$ die oben angegebenen Bedeutungen hat.

Die Verbindungen der Formel (VI) sind allgemein bekannte Verbindungen der organischen Chemie.

Als Beispiele für die Verbindungen der Formel (VI) seien genannt:
Methylchlorid, Methylbromit, Methyliodid, Ethylbromid, Ethyliodid, n-Propylbromid, n-Propyliodid, i-Propylbromid, i-Propyliodid, n-Butylbromid, n-Butyliodid, i-Butylbromid, i-Butyliodid, sec.-Butylbromid, sec.-Butyliodid, tert.-Butylbromid und tert.-Butyliodid, sowie Dimethylsufat, Diethylsulfat und Di-n-propylsulfat.

Die beim erfindungsgemäßen Verfahren außerdem als Ausgangsstoffe zu verwendenden Amine sind durch die Formel (III) allgemein definiert. In dieser Formel (III) stehen $R^3$, $R^4$, $X^1$, $X^2$ und m vorzugsweise bzw. insbesondere bevorzugt für diejenigen Reste, die bereits im Zusammenhang mit der Beschreibung der erfindungsgemäßen Stoffe der Formel (I) vorzugsweise bzw. insbesondere bevorzugt für diese Substituenten genannt wurden.

Bei der Herstellung von optisch aktiven R-und S-Enantiomeren der 3-Amino-2-cyano-acrylsäureester der Formel (Ib) werden die optisch aktiven R-oder S-Enantiomeren der Amine der Formel (IIIa)

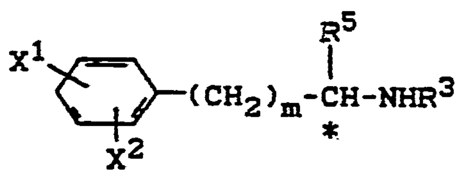

(IIIa)

in welcher
$X^1$, $X^2$, $R^3$, $R^5$ und m die oben angegebenen Bedeutungen haben,
als Ausgangsstoffe eingesetzt. Bevorzugt werden die optisch aktiven S-Enantiomeren der Amine der Formel (IIIa) eingesetzt.

Die Amine der Formel (III) bzw. (IIIa) sind bekannt oder lassen sich nach prinzipiell bekannten Verfahren in einfacher Weise herstellen (vgl. z.B. Organikum, VEB-Verlag Berlin, S. 544 ff. (1977) und Org. Synthesis, Coll. Vol II, 503 (1943)).

Als Beispiele für die Verbindungen der Formel (III) seien genannt:

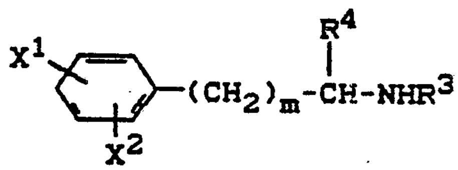 (III)

Tabelle 4

| 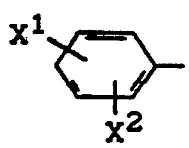 | m | $R^3$ | $R^4$ |
|---|---|---|---|
| 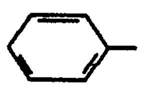 | 0 | H | H |
| 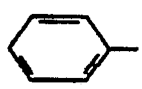 | 0 | H | $CH_3$ |

Tabelle 4 - Fortsetzung

| 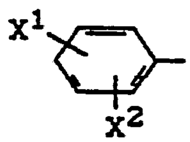 | m | $R^3$ | $R^4$ |
|---|---|---|---|
| 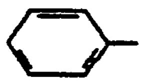 | 0 | $CH_3$ | $CH_3$ |
| 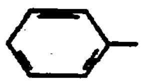 | 0 | $CH_3$ | H |
| 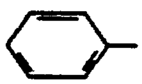 | 1 | H | H |
| 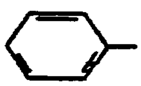 | 1 | H | $CH_3$ |
| 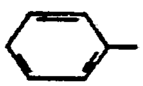 | 1 | $CH_3$ | H |
| 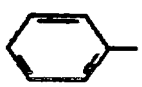 | 1 | $CH_3$ | $CH_3$ |
| 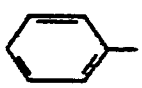 | 1 | $CH_3$ | H |
| 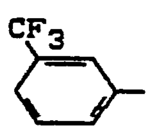 | 1 | H | $CH_3$ |
| 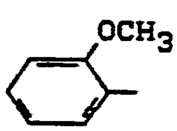 | 1 | H | $CH_3$ |

Tabelle 4 - Fortsetzung

| 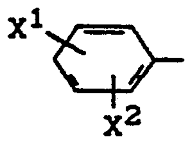 | m | $R^3$ | $R^4$ |
|---|---|---|---|
| 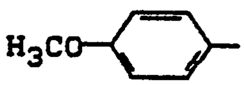 | 1 | H | $CH_3$ |
| 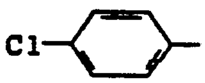 | 1 | H | $CH_3$ |
| 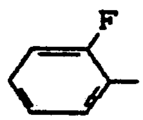 | 1 | H | $CH_3$ |
| 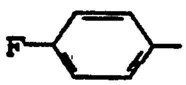 | 1 | H | $CH_3$ |
| 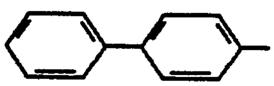 | 0 | H | $CH_3$ |
| 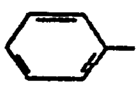 | 2 | H | $CH_3$ |
| 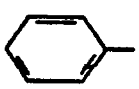 | 3 | H | $CH_3$ |
| 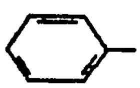 | 4 | H | $CH_3$ |
| 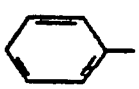 | 5 | H | $CH_3$ |

Tabelle 4 - Fortsetzung

| 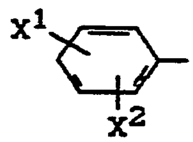 | m | $R^3$ | $R^4$ |
|---|---|---|---|
| 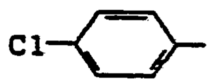 | 1 | H | H |
| 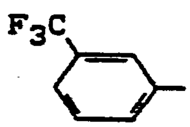 | 1 | H | H |
| 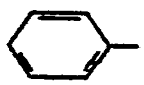 | 0 | H | $C_2H_5$ |
| 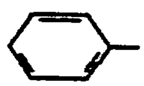 | 0 | H | $C_3H_7$-n |
| 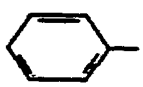 | 0 | H | $C_4H_9$-n |
| 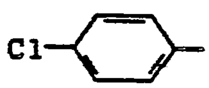 | 0 | H | $CH_3$ |
| 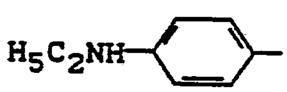 | 0 | H | $CH_3$ |
| 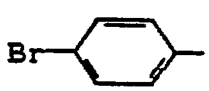 | 0 | H | $CH_3$ |
| 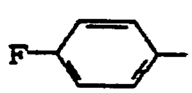 | 0 | H | $CH_3$ |

<u>Tabelle 4</u> - Fortsetzung

| 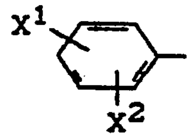 | m | $R^3$ | $R^4$ |
|---|---|---|---|
| 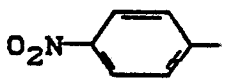 | 0 | H | $CH_3$ |
| 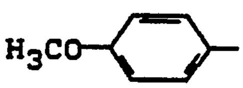 | 0 | H | $CH_3$ |
| 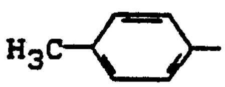 | 0 | H | $CH_3$ |
| 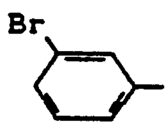 | 0 | H | $CH_3$ |
| 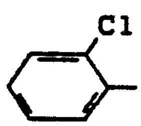 | 0 | H | $CH_3$ |
| 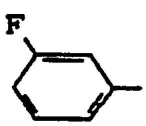 | 0 | H | $CH_3$ |
| 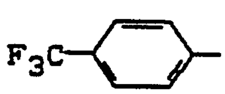 | 0 | H | $CH_3$ |
| 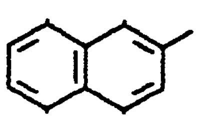 | 0 | H | $CH_3$ |
| 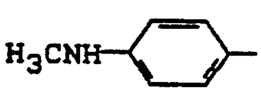 | 0 | H | $CH_3$ |

Tabelle 4 - Fortsetzung

| 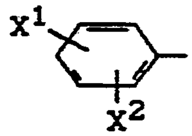 | m | $R^3$ | $R^4$ |
|---|---|---|---|
| 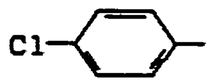 | 0 | H | H |
| 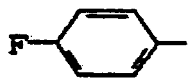 | 0 | H | H |
| 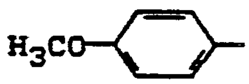 | 0 | H | H |
| 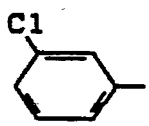 | 0 | H | H |
| 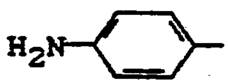 | 0 | H | $CH_3$ |
| 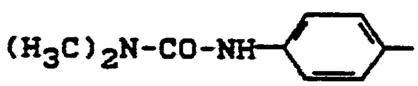 | 0 | H | $CH_3$ |
| 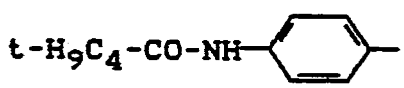 | 0 | H | $CH_3$ |

Als Beispiele für die R-und S-Enantiomeren der Amine der Formel (IIIa) seien genannt:

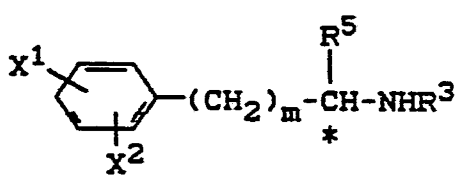

(IIIa)

Tabelle 5

| 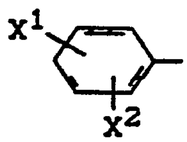 | m | $R^3$ | $R^5$ |
|---|---|---|---|
| 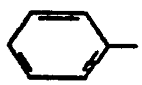 | 0 | H | $CH_3$ |
| 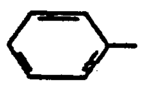 | 0 | H | $C_2H_5$ |
| 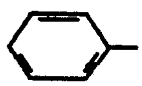 | 0 | H | $n\text{-}C_3H_7$ |
| 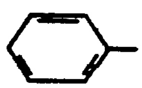 | 0 | H | $i\text{-}C_3H_7$ |
| 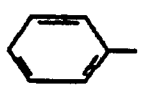 | 0 | H | $n\text{-}C_4H_9$ |
| 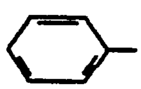 | 0 | H | $i\text{-}C_4H_9$ |
| 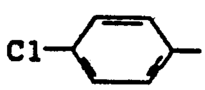 | 0 | H | $CH_3$ |
| 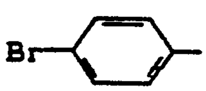 | 0 | H | $CH_3$ |
| 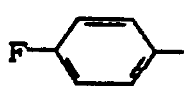 | 0 | H | $CH_3$ |
| 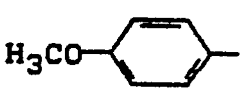 | 0 | H | $CH_3$ |

Tabelle 5 - Fortsetzung

| 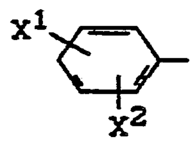 | m | $R^3$ | $R^5$ |
|---|---|---|---|
| 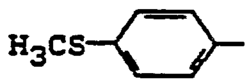 | 0 | H | $CH_3$ |
| 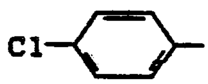 | 1 | H | $CH_3$ |
| 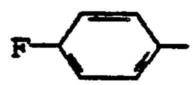 | 1 | H | $CH_3$ |
| 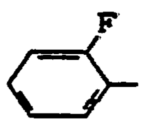 | 1 | H | $CH_3$ |
| 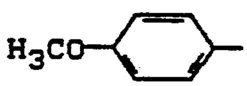 | 1 | H | $CH_3$ |
| 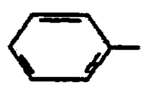 | 2 | H | $CH_3$ |
| 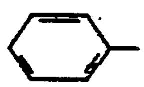 | 3 | H | $CH_3$ |
| 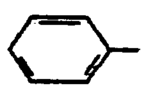 | 4 | H | $CH_3$ |
| 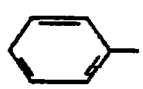 | 5 | H | $CH_3$ |
| 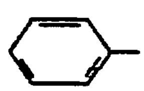 | 0 | $CH_3$ | $CH_3$ |

**Tabelle 5** - Fortsetzung

| 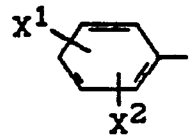 | m | $R^3$ | $R^5$ |
|---|---|---|---|
| 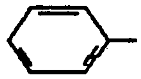 | 0 | H | $C_2H_5$ |
| 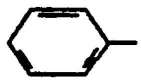 | 0 | H | $n\text{-}C_3H_7$ |
| 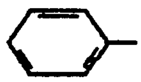 | 0 | H | $i\text{-}C_3H_7$ |
| 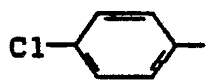 | 0 | H | $C_2H_5$ |
| 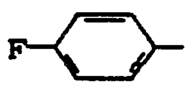 | 0 | H | $C_2H_5$ |
| 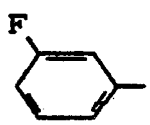 | 0 | H | $C_2H_5$ |
| 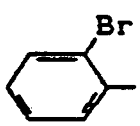 | 0 | H | $C_2H_5$ |
| 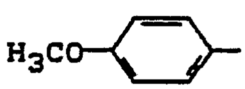 | 0 | H | $C_2H_5$ |
| 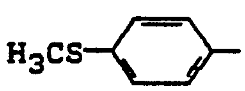 | 0 | H | $C_2H_5$ |

Das erfindungsgemäße Verfahren zur Herstellung der neuen 3-Amino-2-Cyano-acrylsäureester der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykolmonoethylether, Glykoldimethylether und Diglykoldimethylether, Tetrahydrofuran und Dioxan, Alkohole wie Methanol, Ethanol, n-Propanol oder n-Butanol, Amide wie z. B. Dimethylformamid, sowie Dimethylsulfoxid. Bevorzugt wird in Gegenwart von Alkoholen der Formel R'OH gearbeitet, um Nebenreaktionen zu vermeiden.

Die Reaktionstemperaturen können bei dem erfindungsgemäßen Verfahren in einem größeren Bereich variiert werden. Im allgemeinen arbeitet man bei Temperaturen zwischen 0 °C und 120 °C, vorzugsweise bei Temperaturen zwischen 15 °C und 80 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens werden die jeweils benötigten Ausgangsstoffe der Formeln (II) und (III) im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden.

Die neuen Verbindungen fallen in Form von Ölen an, die sich zum Teil nicht unzersetzt destillieren lassen, jedoch durch sogenanntes "Andestillieren", d.h. durch längeres Erhitzen unter vermindertem Druck auf mäßig erhöhte Temperaturen von den letzten flüchtigen Anteilen befreit und auf diese Weise gereinigt werden. Man kann die "andestillierten" Öle jedoch auch in inerten Lösungsmittel wie z.B. n-Hexan oder Benzin aufnehmen, dieses Gemisch über eine Kieselgelsäule filtrieren und anschließend das Lösungsmittel im Wasserstrahlvakuum entfernen. Zu ihrer Charakterisierung dient der Brechungsindex oder die $^1$H-NMR-Spektren.

Die erfindungsgemäßen Wirkstoffe Können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dikotyle Kulturen der Gattungen:</u> Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u> Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Die erfindungsgemäßen Wirkstoffe eignen sich besonders gut zur selektiven Bekämpfung mono-und dikotyler Unkräuter, insbesondere in monokotylen Kulturen im Nachauflaufverfahren. Die erfindungsgemäßen Verbindungen zeigen z.B. bei der Nachauflaufanwendung im Weizen keine Schädigung der Kulturpflanzen, jedoch eine deutliche Wirksamkeit gegen Unkräuter wie Chenopodium, Abutilon, Viola, Polygonum, Echinochloa und Setaria.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder - schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsufonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen können bekannte Herbizide verwendet werden wie z. B. N-(2-Benzthiazolyl)-N,N'-dimethylharnstoff, 3-(3-Chlor-4-methylphenyl)-1,1-dimethylharnstoff, 3-(4-Isopropylphenyl)-1,1-dimethylharnstoff, 2-Chlor-N-{[4-methoxy-6-methyl-1,3,5-triazin-2-yl)-amino]-carbonyl}-benzolsulfonamid, 2-Ethylamino-6-(1,1-dimethylethyl-amino)-4-methylthio-1,3,5-triazin, 2-Chlor-4-ethylamino-6-(1-methylethyl)-1,3,5-triazin, 2-Chlor-4,6-diethylamino-1,3,5-triazin, 2-Chlor-4-(1-cyano-1-methylethylamino)-6-ethylamino-1,3,5-triazin, 4-Amino-6-(1,1-dimethylethyl)-3-methylthio-1,2,4-triazin-5(4H)-on, 4-Amino-6-(1,1-dimethylethyl)-3-ethylthio-1,2,4-triazin-5(4H)-on, 1-Amino-6-ethylthio-3-(2,2-dimethylpropyl)-1,3,5-triazin-2,4-(1H,3H)-dion, 3-(2,4-Dichlor-phenoxy)-6-nitro-benzoesäuremethylester, 2-[4-(2,4-Dichlorphenoxy)-phenoxy]-propionsäuremethylester, 2-{4-[(6-Chlor-2-benzoxazolyl)-oxy]-phenoxy}-propionsäureethylester, das R-Enantiomere des 2-{4-[(3,5-Dichlor-2-pyridyl)oxy]-phenoxy}-propionsäure-(trimethylsilyl)-methylesters, 2,4-Dichlorphenoxyessigsäure, 2-(2,4-Dichlorphenoxy)-propionsäure, 4-Chlor-2-methyl-phenoxy-essigsäure, 2-(4-Chlor-2-methyl-phenoxy)-propionsäure, 3,5-Diiod-4-hydroxy-benzonitril, 3,5-Dibrom-4-hydroxy-benzonitril, 0-(6-Chlor-3-phenyl-pyridazin-4-yl)-S-octyl-thiokohlensäureester, [(4-Amino-3,5-dichlor-6-fluor-2-pyridyl)oxy]-essigsäure und-3-Isopropyl-2,1,3-benzothiadiazinon-(4)-2,2-dioxid. Einige Mischungen besitzen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Bevorzugt wird nach dem Auflaufen appliziert.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele

### Beispiel 1

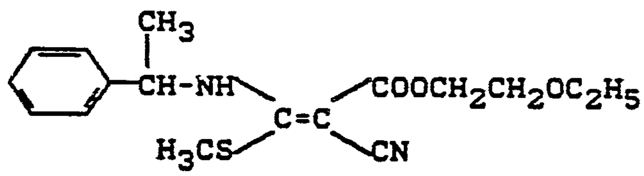

Zu einer Lösung von 26,1 g (0,1 Mol) 2-Cyano-3,3-dimethylthio-acrylsäureethoxyethylester in 150 ml Ethanol werden 12,1 g (0,1 Mol) 1-Phenyl-ethylamin so zugetropft, daß die Innentemperatur von +35 °C nicht überschritten wird. Anschließend wird das Lösungsmittel entfernt. Das erhaltene ölige Rohprodukt wird mit n-Hexan verrührt und über eine Kieselgelsäule gereinigt.

Man erhält so 16,7 g (50 % der Theorie) 2-Cyano-3-methylthio-3-(1-phenyl-ethylamino)-acrylsäure-2-(ethoxy)-ethylester als gelbes Öl.

$^1$H-NMR (CDCl$_3$): $\delta$ = 5,25 (m, 1H, -CH-N-), 1,6 (d, 3H, $CH_3$ -C-Aryl) ppm.

### Beispiel 2

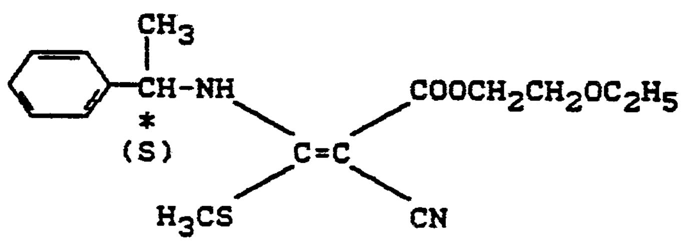

Zu einer Lösung von 26,1 g (0,1 Mol) 2-Cyano-3,3-dimethylthio-acrylsäureethoxyethylester in 150 ml Ethanol werden 12,1 g (0,1 Mol) (S-)-1-Phenyl-ethylamin so zugetropft, daß die Innentemperatur von +35 °C nicht überschritten wird. Anschließend wird das Lösungsmittel entfernt. Das erhaltene ölige Rohprodukt wird mit n-Hexan verrührt und über eine Kieselgelsäule gereinigt.

Man erhält so 16,7 g (50 % der Theorie) (S+)-2-Cyano-3-methylthio-3-(1-phenyl-ethylamino)-acrylsäure-2-(ethoxy) -ethylester als gelbes Öl.

Drehwert: $[\alpha]_D^{28,5}$ = +192°

(1-molare Lösung in Chloroform; Küvettenlänge 10 cm)

Analog Beispiel 1 oder 2 und gemäß den allgemeinen Angaben zur Herstellung können die folgenden Verbindungen der Formel (I) hergestellt werden:

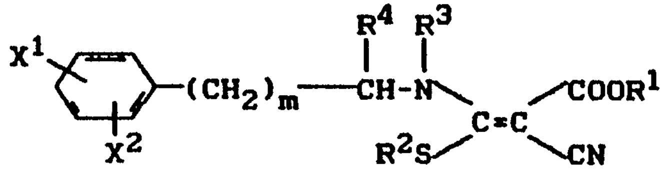

(I)

Tabelle 6

| Beisp.-Nr. | 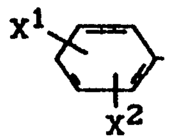 | $R^1$ | $R^2$ | $R^3$ | $R^4$ | m | Phys. Konst. $^1$H-NMR ($CDCl_3$) /[ppm.] |
|---|---|---|---|---|---|---|---|
| 3 | 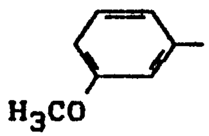 | $-CH_2CH_2OC_2H_5$ | $CH_3$ | H | H | 0 | δ = 4,7 (d,2H, $-CH_2-N-$) |
| 4** | 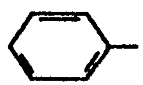 | $-CH_2CH_2OC_2H_5$ | $CH_3$ | H | $CH_3$ | 0 | δ = 5,3 (m, 1H, -CH-N-) |
| 5 | 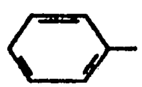 | $-CH_2CH_2OC_2H_5$ | $i-C_3H_7$ | H | $CH_3$ | 0 | δ = 4,0 (m,1H, -S-CH-) |
| 6** | 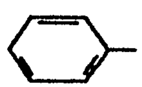 | $-CH_2CH_2OC_2H_5$ | $i-C_3H_7$ | H | $CH_3$ | 0 | δ = 4,0 (m, 1H, -S-CH-) |

**Tabelle 6** - Fortsetzung

| Beisp.-Nr. | 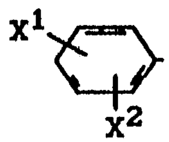 | $R^1$ | $R^2$ | $R^3$ | $R^4$ | m | Phys. Konst. $^1H$-NMR ($CDCl_3$) /[ppm.] |
|---|---|---|---|---|---|---|---|
| 7* | 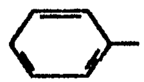 | $-CH_2CH_2OC_2H_5$ | i-$C_3H_7$ | H | $CH_3$ | 0 | δ = 4,0 (m,1H,<br>/<br>-S-CH-) |
| 8 | 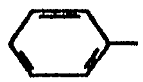 | $-CH_2CH_2OC_2H_5$ | $C_2H_5$ | H | $CH_3$ | 0 | δ = 3,1 (m,2H,<br>$-S-CH_2-$) |
| 9** | 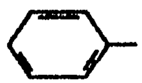 | $-CH_2CH_2OC_2H_5$ | $C_2H_5$ | H | $CH_3$ | 0 | δ = 3,1 (m,2H,<br>$-S-CH_2-$) |
| 10* | 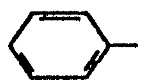 | $-CH_2CH_2OC_2H_5$ | $C_2H_5$ | H | $CH_3$ | 0 | δ = 3,1 (m,2H,<br>$-S-CH_2-$) |
| 11 | 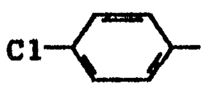 | $-CH_2CH_2OC_2H_5$ | $CH_3$ | H | $CH_3$ | 0 | δ = 2,6 (s,3H,<br>$-S-CH_3$) |
| 12 | 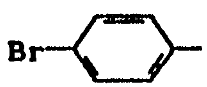 | $-CH_2CH_2OC_2H_5$ | $CH_3$ | H | $CH_3$ | 0 | δ = 2,6 (s,3H,<br>$-S-CH_3$) |
| 13 | 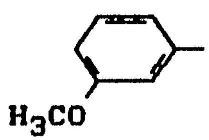 | $-CH_2CH_2OC_2H_5$ | $CH_3$ | H | $CH_3$ | 0 | δ = 10,3 (d,1H,<br>/<br>-NH) |

Tabelle 6 - Fortsetzung

| Beisp.-Nr. | 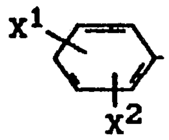 | $R^1$ | $R^2$ | $R^3$ | $R^4$ | m | Phys. Konst. $^1H$-NMR ($CDCl_3$) /[ppm.] |
|---|---|---|---|---|---|---|---|
| 14 | 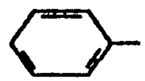 | $-CH_2CH_2OC_2H_5$ | $CH_3$ | $CH_3$ | H | 0 | δ = 4,9 (s,2H, $-CH_2-N-$) |
| 15 | 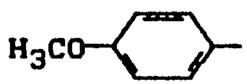 | $-CH_2CH_2OC_2H_5$ | $CH_3$ | H | H | 0 | δ = 3,8 (m,2H, $-CH_2-N-$) |
| 16 | 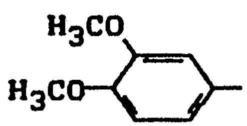 | $-CH_2CH_2OC_2H_5$ | $CH_3$ | H | H | 0 | δ = 3,8 (m,2H, $-CH_2-N-$) |
| 17 | 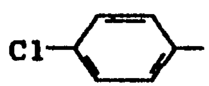 | $-CH_2CH_2OC_2H_5$ | $CH_3$ | H | H | 0 | δ = 4,8 (d,2H, $-CH_2-N-$) |
| 18 | 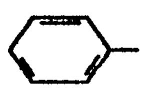 | $-CH_2CH_2OC_2H_5$ | $CH_3$ | H | H | 1 | δ = 2,9 (t,2H, $-CH_2-Ar$) |
| 19 | 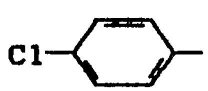 | $-CH_2CH_2OC_2H_5$ | $CH_3$ | H | H | 1 | δ = 2,9 (t,2H, $-CH_2-Ar$) |

**<u>Tabelle 6</u> - Fortsetzung**

| Beisp.-Nr. | 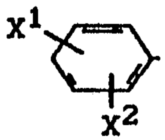 | $R^1$ | $R^2$ | $R^3$ | $R^4$ | m | Phys. Konst. $^1H$-NMR ($CDCl_3$) /[ppm.] |
|---|---|---|---|---|---|---|---|
| 20 | 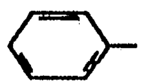 | $-CH_2CH_2OC_2H_5$ | $CH_3$ | H | H | 2 | $\delta$ = 2,7 (t,2H, Ar-C-$CH_2$-) |
| 21 | 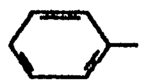 | $-CH_2CH_2OC_2H_5$ | $CH_3$ | H | $CH_3$ | 2 | $\delta$ = 2,6 (m,2H, Ar-C-$CH_2$-) |
| 22 | 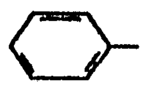 | $-CH_2CH_2OC_2H_5$ | $CH_3$ | H | H | 3 | $\delta$ = 1,7 (m,2H, $-CH_2$-C-N-) |
| 23 | 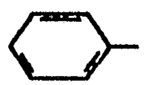 | $-CH_2CH_2OC_3H_7$-i | $CH_3$ | H | $CH_3$ | 0 | $\delta$ = 2,6 (s,3H, $-SCH_3$) |
| 24* | 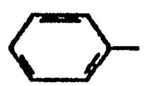 | $-CH_2CH_2OC_3H_7$-i | $CH_3$ | H | $CH_3$ | 0 | $\delta$ = 2,6 (s,3H, $-SCH_3$) |
| 25** | 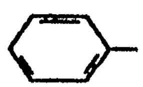 | $-CH_2CH_2OC_3H_7$-i | $CH_3$ | H | $CH_3$ | 0 | $\delta$ = 2,6 (s,3H, $-SCH_3$) |

**Tabelle 6** - Fortsetzung

| Beisp.-Nr. | 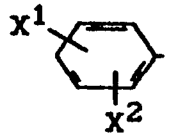 | $R^1$ | $R^2$ | $R^3$ | $R^4$ | m | Phys. Konst. $^1H$-NMR ($CDCl_3$) /[ppm.] |
|---|---|---|---|---|---|---|---|
| 26 | 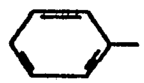 | $-CH_2CH_2OCH_2-$ 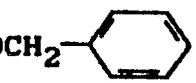 | $CH_3$ | H | $CH_3$ | 0 | δ = 3,7 (q,2H, $-OCH_2-C-$) |
| 27* | 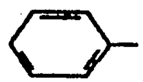 | $-CH_2CH_2OCH_2-$ 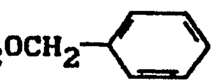 | $CH_3$ | H | $CH_3$ | 0 | δ = 3,7 (q,2H, $-OCH_2-C-$) |
| 28 | 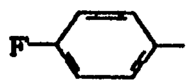 | $-CH_2CH_2OC_2H_5$ | $CH_3$ | H | $CH_3$ | 0 | δ = 5,3 (quint, 1H, Ar-CH-) |
| 29 | 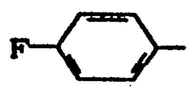 | $-CH_2CH_2OC_2H_5$ | $CH_3$ | H | H | 0 | δ = 4,8 (d,2H, $-CH_2-N-$) |
| 30 | 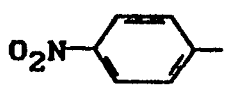 | $-CH_2CH_2OC_2H_5$ | $CH_3$ | H | H | 0 | δ = 2,6 (s,3H, $-SCH_3$) |
| 31 | 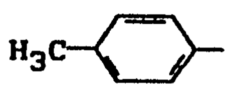 | $-CH_2CH_2OC_2H_5$ | $CH_3$ | H | $CH_3$ | 0 | δ = 2,6 (s,3H, $-SCH_3$) |

<u>Tabelle 6</u> - Fortsetzung

| Beisp.-Nr. | 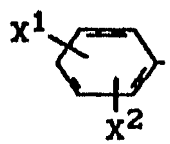 | $R^1$ | $R^2$ | $R^3$ | $R^4$ | m | Phys. Konst. $^1H$-NMR ($CDCl_3$) /[ppm.] |
|---|---|---|---|---|---|---|---|
| 32 | 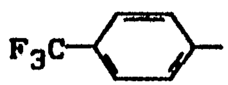 | $-CH_2CH_2OC_2H_5$ | $CH_3$ | H | H | 0 | δ = 4,9 (d,2H, -CH-N-) |
| 33** | 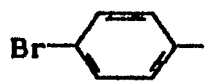 | $-CH_2CH_2OC_2H_5$ | $CH_3$ | H | $CH_3$ | 0 | δ = 2,6 (s,3H, $-S-CH_3$) |
| 34 | 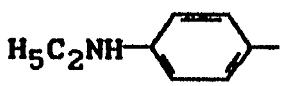 | $-CH_2CH_2OC_2H_5$ | $CH_3$ | H | $CH_3$ | 0 | δ = 5,3 (quint, 1H, -CH-N-) |
| 35 | 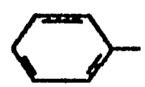 | $-CH_2CH_2OC_2H_5$ | $CH_3$ | H | $n-C_4H_9$ | 0 | δ = 1,9 (m,2H, $-C-CH_2-C-$) |
| 36 | 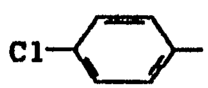 | $-CH_2CH_2OC_2H_5$ | $CH_3$ | H | H | 1 | δ = 4,3 (t,2H, $-CH_2-N-$) |
| 37 | 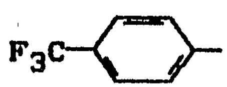 | $-CH_2CH_2OC_2H_5$ | $CH_3$ | H | $CH_3$ | 1 | δ = 2,7 (t,2H, $-CH_2-N-$ |

**Tabelle 6** - Fortsetzung

| Beisp.-Nr. | 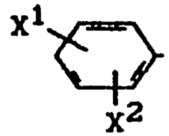 | $R^1$ | $R^2$ | $R^3$ | $R^4$ | m | Phys. Konst. $^1H$-NMR ($CDCl_3$) /[ppm.] |
|---|---|---|---|---|---|---|---|
| 38 | 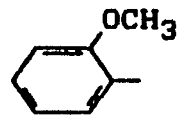 | $-CH_2CH_2OC_2H_5$ | $CH_3$ | H | H | 0 | δ = 4,8 (d,2H, / $-CH_2-N-$) |
| 39 | 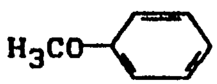 | $-CH_2CH_2OC_2H_5$ | $CH_3$ | H | H | 1 | δ = 3,8 (m,2H, $Ar-CH_2-$) |
| 40 | 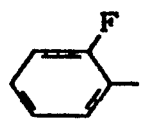 | $-CH_2CH_2OC_2H_5$ | $CH_3$ | H | H | 0 | δ = 4,8 (d,2H, / $-CH_2-N-$) |
| 41 | 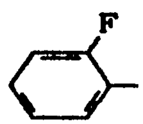 | $-CH_2CH_2OC_2H_5$ | $CH_3$ | H | $CH_3$ | 0 | δ = 2,6 (s,3H, $-SCH_3$) |
| 42 | 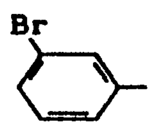 | $-CH_2CH_2OC_2H_5$ | $CH_3$ | H | $CH_3$ | 0 | δ = 1,6 (d,3H, / $-C-CH_3$ /) |

**Tabelle 6** - Fortsetzung

| Beisp.-Nr. | 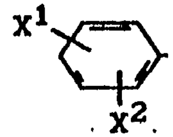 | $R^1$ | $R^2$ | $R^3$ | $R^4$ | m | Phys. Konst. $^1H$-NMR ($CDCl_3$) /[ppm.] |
|---|---|---|---|---|---|---|---|
| 43 | 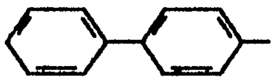 | $-CH_2CH_2OC_2H_5$ | $CH_3$ | H | $CH_3$ | 0 | δ = 2,6 (s,3H, $-SCH_3$) |
| 44 | 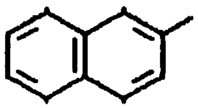 | $-CH_2CH_2OC_2H_5$ | $CH_3$ | H | $CH_3$ | 0 | δ = 2,6 (s,3H, $-SCH_3$) |
| 45 | 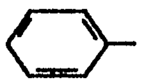 | $-CH_2CH_2OC_2H_5$ | $CH_3$ | H | $CH_3$ | 1 | δ = 2,8 (s,1H, / $-N-CH_3$) |
| 46 | 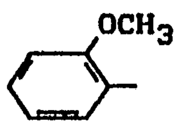 | $-CH_2CH_2OC_2H_5$ | $CH_3$ | H | H | 1 | δ = 10,1 (m,1H, / -NH) |
| 47 | 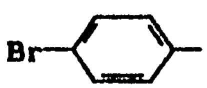 | $-CH_2CH_2OC_2H_5$ | $CH_3$ | H | H | 1 | δ = 3,9 (q,2H, / $-CH_2-N-$) |
| 48 | 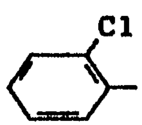 | $-CH_2CH_2OC_2H_5$ | $CH_3$ | H | H | 0 | δ = 4,5 (d,2H, / $-CH_2-N-$) |

**Tabelle 6** - Fortsetzung

| Beisp.-Nr. | 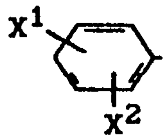 | $R^1$ | $R^2$ | $R^3$ | $R^4$ | m | Phys. Konst. $^1H$-NMR ($CDCl_3$) /[ppm.] |
|---|---|---|---|---|---|---|---|
| 49 | 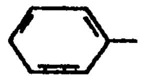 | $-CH_2CH_2OC_2H_5$ | $CH_3$ | H | H | 0 | δ = 2,6 (s,3H, $-SCH_3$) |
| 50 | 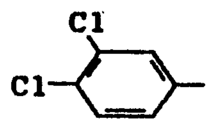 | $-CH_2CH_2OC_2H_5$ | $CH_3$ | H | H | 0 | δ = 4,7 (d,2H, / $-CH_2-N-$) |
| 51 | 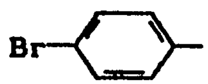 | $-CH_2CH_2OC_2H_5$ | $CH_3$ | H | H | 0 | δ = 4,7 (d,2H, / $-CH_2-N-$) |
| 52 | 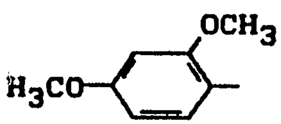 | $-CH_2CH_2OC_2H_5$ | $CH_3$ | H | H | 0 | δ = 4,6 (d,2H, / $-CH_2-N-$) |
| 53 | 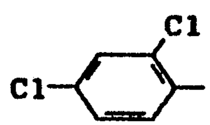 | $-CH_2CH_2OC_2H_5$ | $CH_3$ | H | H | 0 | δ = 4,9 (d,2H, / $-CH_2-N-$) |
| 54 | 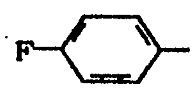 | $-CH_2CH_2OC_2H_5$ | $CH_3$ | H | H | 1 | δ = 10,1 (m,1H, / -NH) |

Beispiel 5 A

Das Strukturisomere von Beispiel 5

$^1$H-NMR ($CDCl_3$) : $\delta$ = 9,65 (d, 1H, -NH), 4,8( q, 1H, -CH-N-), 4,5 (m, 1H, -S-CH-) ppm.

Beispiel 6 A

Das Stukturisomere von Beispiel 6

$^1$H-NMR ($CDCl_3$) : $\delta$ = 9,65 (d, 1H, -NH), 4,8 (q, 1H, -CH-N-), 4,5 (m, 1H, -S-CH-) ppm.

Beispiel 7 A

Das Stukturisomere von Beispiel 7

$^1$H-NMR ($CDCl_3$) : $\delta$ = 9,65 (d, 1H, -NH), 4,8 (q, 1H, -CH-N-), 4,5 (m, 1H, -S-CH-) ppm.

Bemerkung:
* = das optisch aktive S-enantiomere Derivat
** = das optisch aktive R-enantiomere Derivat

Ausgangsverbindungen der Formel (V)

Beispiel (V-1)

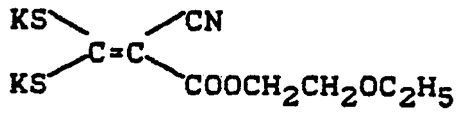

Eine Lösung von 314 g (2 Mol) 2-Cyanoessigsäure-(2-ethoxyethylester und 152 g (2 Mol) Kohlenstoffdisulfid in 3 l Ether (abs.) wird bei 10 °C mit 470 g (4 Mol) 95 %iger Kalium-tert.-butanolat versetzt. Nach beendeter Zugabe wird das Reaktionsgemisch noch 30 Min. bei 20 °C gerührt. Das Dikaliumsalz der oben angegebenen Strukturformel wird abgenutscht und ohne weitere Reinigung für die Herstellung der Verbindungen der Formel (II) verwendet.

Analog Beispiel (V-1) können die folgenden Verbindungen der Formel (V) hergestellt werden:

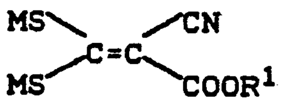 (V)

**Tabelle 7**

| Beisp. Nr. | M | $R^1$ |
|---|---|---|
| (V-2) | K | $-CH_2CH_2OCH_3$ |
| (V-3) | K | $-CH_2CH_2OC_3H_7$-n |
| (V-4) | K | $-CH_2CH_2OC_3H_7$-i |
| (V-5) | K | $-CH_2CH_2OCH_2-$ 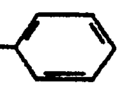 |

Ausgangsverbindungen der Formel (II)

Beispiel (II-1)

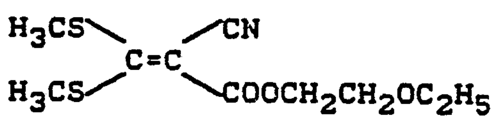

Das Dikaliumsalz von Beispiel (V-1) wird in 2000 ml Wasser gelöst und unter Kühlung tropfenweise bei einer Innentemperatur von 20 °C mit 504 g (4 Mol) Dimethylsulfat umgesetzt. Nach Beendigung der Zugabe wird eine halbe Stunde bei 20 °C nachgerührt. Die wässrige Phase wird abgetrennt und die organische Phase in 2000 ml Ether aufgenommen. Die Etherphase wird viermal mit jeweils 1000 ml Wasser gewaschen, über Natriumsulfat getrocknet und eingeengt. Der Rückstand wird in n-Hexan aufgenommen und über eine Kieselgelsäule gereinigt. Das Rohprodukt kann jedoch auch ohne weitere Reinigung eingesetzt werden.

Man erhält so 222 g (85 % der Theorie) 2-Cyano-3,3-dimethylthio-acrylsäure-(2-ethoxy)-ethylester als gelbes Öl.

$^1H$-NMR ($CDCl_3$) : $\delta$ = 4,4 (m, 2H, $-COOCH_2-$),

1,2 (t, 3H, $-O-\overset{|}{\underset{|}{C}}-CH_3$) ppm.

Analog Beispiel (II-1) können die folgenden Verbindungen der Formel (II) hergestellt werden.

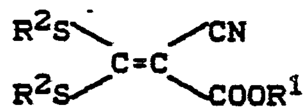
(II)

**Tabelle 8**

| Beisp.-Nr. | $R^1$ | $R^2$ | Phys. Konst. $^1H$-NMR ($CDCl_3$) /[ppm.] |
|---|---|---|---|
| II-2 | $-CH_2CH_2OC_2H_5$ | $-C_2H_5$ | $\delta$ = 3,1 (m, 4H, $-S-CH_2-$) |
| II-3 | $-CH_2CH_2OC_2H_5$ | $-C_3H_7$-i | $\delta$ = 1,4 (m,12H, / $-S(CH_3)_2$ ) |
| II-4 | $-CH_2CH_2OC_2H_5$ | $-C_4H_9$-i | |
| II-5 | $-CH_2CH_2OC_2H_5$ | $-CH_2CH{=}CH_2$ | |
| II-6 | $-CH_2CH_2OCH_2-$ 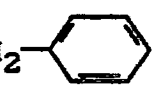 | $-CH_3$ | |

Beispiel A

Post-emergence-Test / Gewächshaus

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)

100 % = totale Vernichtung

Bei diesem Test zeigen z.B. die Verbindungen der Herstellungsbeispiele (1) und (2) bei voller Verträglichkeit in Weizen eine wesentlich bessere Wirksamkeit gegen mono-und dikotyle Unkräuter wie z.B. Abutilon, Viola, Echinochloa usw. als die Vergleichssubstanz (A).

## Ansprüche

1. 3-Amino-2-cyano-acrylsäureester der Formel (I)

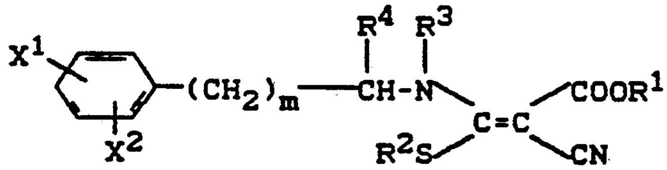

(I)

in welcher
$R^1$ für Alkoxyalkyl oder Benzyloxyalkyl steht,
$R^2$ für Alkyl oder Alkenyl steht,
$R^3$ für Wasserstoff oder Alkyl steht,
$R^4$ für Wasserstoff oder für gegebenenfalls durch Hydroxy, Halogen, Alkoxy oder Dialkylamino substituiertes Alkyl steht,
$X^1$ und $X^2$ gleich oder verschieden sind und für Wasserstoff, Halogen, Nitro, Alkyl, Alkoxy, Alkylthio, Halogenalkyl, Halogenalkoxy, Amino, Alkylamino, Alkylcarbonylamino, N-Alkylcarbonyl-N-alkylamino, Dialkylaminocarbonylamino oder jeweils gegebenenfalls substituiertes Aryl oder Aryloxy stehen,
oder
$X^1$ und $X^2$ gemeinsam mit dem angrenzenden Phenylrest für Naphthyl stehen und
m für die Zahlen 0, 1, 2, 3, 4 oder 5 steht,
sowie für den Fall, daß $R^4$ nicht für Wasserstoff steht, auch deren optisch aktive Verbindungen.

2. 3-Amino-2-cyanoacrylsäureester der Formel (I) gemäß Anspruch 1, in welcher
$R^1$ für Alkoxyalkyl mit jeweils 1 bis 6 Kohlenstoffatomen in den einzelnen Alkylteilen oder für Benzyloxyalkyl mit 1 bis 6 Kohlenstoffatomen im Alkylteil steht,
$R^2$ für Alkyl mit 1 bis 6 Kohlenstoffatomen oder für Alkenyl mit 3 bis 8 Kohlenstoffatomen steht,
$R^3$ für Wasserstoff oder Alkyl mit 1 bis 6 Kohlenstoffatomen steht,
$R^4$ für Wasserstoff oder für gegebenenfalls durch Hydroxy, Fluor, Chlor, $C_1$-$C_4$-Alkoxy oder Di-$C_1$-$C_4$-alkylamino substituiertes Alkyl mit 1 bis 6 Kohlenstoffatomen steht,
$X^1$ und $X^2$ gleich oder verschieden sind und für Wasserstoff, Fluor, Chlor, Brom, Nitro, $C_1$-$C_4$-Alkyl, $C_1$-$C_4$-Alkoxy, $C_1$-$C_4$-Alkylthio, Halogen-$C_1$-$C_4$-alkyl, Halogen-$C_1$-$C_4$-alkoxy,
Amino, $C_1$-$C_4$-Alkylamino, $C_1$-$C_4$-Alkylcarbonylamino, N-$C_1$-$C_4$-Alkylcarbonyl-N-$C_1$-$C_4$-alkylamino, Di-$C_1$-$C_4$-alkylaminocarbonylamino oder für jeweils gegebenenfalls einfach bis dreifach, gleich oder verschieden durch Halogen, wie Fluor, Chlor und Brom, Nitro, Cyano, $C_1$-$C_2$-Alkyl, $C_1$-$C_2$-Alkoxy, $C_1$-$C_2$-Alkylthio, Trifluormethyl, Trifluormethoxy oder Trifluormethylthio substituiertes Aryl oder Aryloxy mit jeweils 6 bis 10 Kohlenstoffatomen im Arylteil stehen, oder
$X^1$ und $X^2$ gemeinsam mit dem angrenzenden Phenylrest für Naphthyl stehen und
m für die Zahlen 0, 1, 2 oder 3 steht.

3. 3-Amino-2-cyanoacrylsäureester der Formel (I) gemäß Anspruch 1, in welcher
$R^1$ für Alkoxyalkyl mit jeweils 1 bis 4 Kohlenstoffatomen in den einzelnen Alkylteilen oder für Benzyloxyalkyl mit 1 bis 4 Kohlenstoffatomen im Alkylteil steht,
$R^2$ für Alkyl mit 1 bis 4 Kohlenstoffatomen oder Alkenyl mit 3 bis 6 Kohlenstoffatomen steht,
$R^3$ für Wasserstoff oder Alkyl mit 1 bis 4 Kohlenstoffatomen steht,
$R^4$ für Wasserstoff oder für gegebenenfalls durch Hydroxy, Fluor, Chlor, Methoxy, Ethoxy, Dimethylamino oder Diethylamino substituiertes Alkyl mit 1 bis 4 Kohlenstoffatomen steht,
$X^1$ und $X^2$ gleich oder verschieden sind und für Wasserstoff, Fluor, Chlor, Brom, Nitro, Methyl, Ethyl, Methoxy, Ethoxy, Methylthio, Ethylthio, Trifluormethyl, Trifluormethoxy, Amino, Methylamino, Ethylamino, n-Propylamino, i-Propylamino, n-Butylamino, tert.-Butylamino, Methylcarbonylamino, Ethylcarbonylamino, n-Propylcarbonylamino, i-Propylcarbonylamino, n-Butylcarbonylamino, i-Butylcarbonylamino, tert.-Butylcarbonylamino, N-Methylcarbonyl-N-methylamino, N-Ethylcarbonyl-N-methylamino, N-n-Propylcarbonyl-N-methylamino, N-i-Propylcarbonyl-N-methylamino oder für jeweils gegebenenfalls einfach oder zweifach, gleich

oder verschieden durch Fluor, Chlor, Nitro, Cyano, Methyl, Ethyl, Methoxy, Ethoxy, Methylthio, Ethylthio, Trifluormethyl, Trifluormethoxy oder Trifluormethylthio substituiertes Phenyl oder Phenoxy stehen, oder
$X^1$ und $X^2$ gemeinsam mit dem angrenzenden Phenylrest für Naphthyl stehen und
m für die Zahlen 0, 1 oder 2 steht.

4. R-und S-Enantiomere der 3-Amino-2-cyano-acrylsäureester der Formel (Ib)

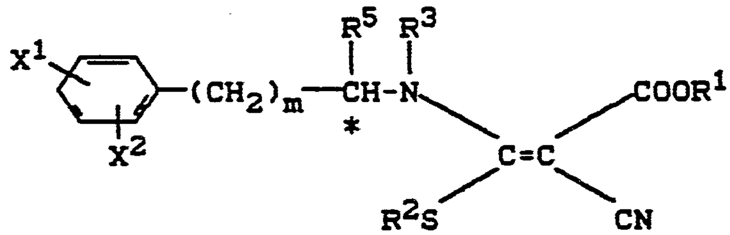
(Ib)

in welcher
$X^1$, $X^2$, $R^1$, $R^2$, $R^3$ und m die in Anspruch 2 angegebenen Bedeutungen haben und in denen das mit dem Stickstoff verbundene, durch (*)gekennzeichnete Kohlenstoffatom ein Chiralitätszentrum darstellt und
$R^5$ für $C_1$-$C_4$-Alkyl steht.

5. Verfahren zur Herstellung von 3-Amino-2-cyano-acrylsäureester der Formel (I)

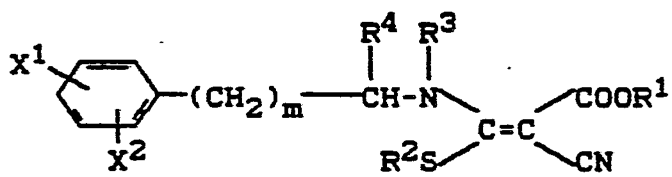
(I)

in welcher
$R^1$ für Alkoxyalkyl oder Benzyloxyalkyl steht,
$R^2$ für Alkyl oder Alkenyl steht,
$R^3$ für Wasserstoff oder Alkyl steht,
$R^4$ für Wasserstoff oder für gegebenenfalls durch Hydroxy, Halogen, Alkoxy oder Dialkylamino substituiertes Alkyl steht,
$X^1$ und $X^2$ gleich oder verschieden sind und für Wasserstoff, Halogen, Nitro, Alkyl, Alkoxy, Alkylthio, Halogenalkyl, Halogenalkoxy Amino, Alkylamino, Alkylcarbonylamino, N-Alkylcarbonyl-N-alkylamino, Dialkylaminocarbonylamino oder jeweils gegebenenfalls substituiertes Aryl oder Aryloxy stehen, oder
$X^1$ und $X^2$ gemeinsam mit dem angrenzenden Phenylrest für Naphthyl stehen und
m für die Zahlen 0, 1, 2, 3, 4 oder 5 steht,
dadurch gekennzeichnet, daß man 2-Cyano-acrylsäureester der Formel (II)

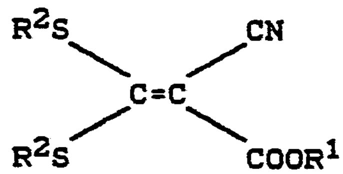
(II)

in welcher
$R^1$ und $R^2$ die oben angegebenen Bedeutungen haben,
mit Aminen der Formel (III)

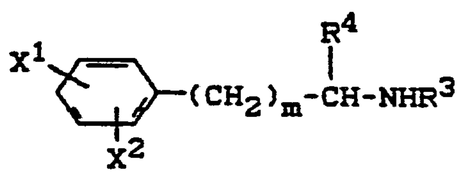
(III)

in welcher-
m, $X^1$, $X^2$, $R^3$ und $R^4$ die oben angegebenen Bedeutungen haben,
gegebenenfalls in Gegenwart von Verdünnungsmitteln umsetzt.

6. Herbizide Mittel, gekennzeichnet durch einen Gehalt an mindestens einem 3-Amino-2-cyano-acrylsäureester der Formel (I) gemäß den Ansprüchen 1 bis 5.

7. Verfahren zur Bekämpfung von Unkräutern, dadurch gekennzeichnet, daß man 3-Amino-2-cyano-acrylsäureester der Formel (I) gemäß den Ansprüchen 1 bis 5 auf die Unkräuter und/oder ihren Lebensraum einwirken läßt.

8. Verwendung von 3-Amino-2-cyano-acrylsäureestern der Formel (I) gemäß den Ansprüchen 1 bis 5 zur Bekämpfung von Unkräutern.

9. Verfahren zur Herstellung von herbiziden Mitteln, dadurch gekennzeichnet, daß man 3-Amino-2-cyano-acrylsäureester der Formel (I) gemäß den Ansprüchen 1 bis 5 mit Streckmitteln und/oder oberflächenaktiven Mitteln vermischt.

10. 2-Cyano-arylsäureester der Formel (II),

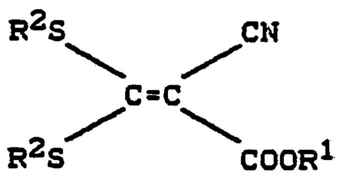

(II)

in welcher
$R^1$ für Alkoxyalkyl oder Benzyloxyalkyl steht und
$R^2$ für Alkyl oder Alkenyl steht.

Europäisches Patentamt

# EUROPÄISCHER RECHERCHENBERICHT

Nummer der Anmeldung

EP 87 10 5007

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US-A-3 406 183 (R.L. LAVAL)<br>--- | 1 | C 07 C 149/243<br>C 07 C 149/20<br>A 01 N 37/44 |
| D,A | US-A-4 201 569 (L.W. HEDRICH)<br>--- | 1 | |
| D,A | US-A-4 154 599 (L.W. HEDRICH)<br>--- | 1 | |
| D,A | CHEMISCHE BERICHTE, Band 95, Nr. 12, 1962, Seiten 2861-2870, Verlag Chemie GMBH, Weinheim; R. GOMPPER et al.: "Substituierte Dithiocarbonsäuren und Ketenmercaptale 2,3"<br>----- | 1 | |
| | | | **RECHERCHIERTE SACHGEBIETE (Int. Cl.4)** |
| | | | C 07 C 149/243<br>C 07 C 149/20 |

Der vorliegende Recherchenbericht wurde für alle Patentansprüche erstellt.

| Recherchenort | Abschlußdatum der Recherche | Prüfer |
|---|---|---|
| BERLIN | 10-07-1987 | KAPTEYN H G |

KATEGORIE DER GENANNTEN DOKUMENTE
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument